# EUROPÄISCHE PATENTSCHRIFT

(11) **EP 2 948 089 B1**
(45) Veröffentlichungstag und Bekanntmachung des Hinweises auf die Patenterteilung: **22.08.2018**
(21) Anmeldenummer: 14700556.5
(22) Anmeldetag: 16.01.2014
(51) Int. Cl.: A47L 15/44, A61B 90/70

(54) **DOSIERUNG EINES AUFBEREITUNGSFLUIDS FÜR CHIRURGISCHE INSTRUMENTE**
METERING OF A PREPARATION FLUID FOR SURGICAL INSTRUMENTS
DOSAGE D'UN FLUIDE DE TRAITEMENT UTILISÉ POUR DES INSTRUMENTS CHIRURGICAUX

(30) Priorität: 23.01.2013 DE 102013201050
(43) Veröffentlichungstag der Anmeldung: 02.12.2015
(73) Patentinhaber: OLYMPUS Winter & Ibe GmbH, 22045 Hamburg (DE)
(72) Erfinder: THATE, Henning, 22417 Hamburg (DE)
(74) Vertreter: Seemann & Partner Patentanwälte mbB
(86) Internationale Anmeldenummer: PCT/EP2014/000116
(87) Internationale Veröffentlichungsnummer: WO 2014/114435

(56) Entgegenhaltungen:
- DE-A1-102009 051 619
- GB-A- 2 111 021
- US-A- 3 370 597
- US-A- 3 749 288
- US-A1- 2009 000 648
- US-A1- 2010 218 790

## Beschreibung

Die Erfindung betrifft eine Dosiereinrichtung für eine Aufbereitungsvorrichtung zum Aufbereiten von chirurgischen Instrumenten, insbesondere Endoskopen. Ferner betrifft die Erfindung eine Verwendung einer derartigen Dosiereinrichtung sowie eine Aufbereitungsvorrichtung zum Aufbereiten von chirurgischen Instrumenten, insbesondere Endoskopen, und ein Verfahren zum Aufbereiten eines chirurgischen Instruments, insbesondere Endoskops, mittels eines Aufbereitungsfluids.

Im Stand der Technik ist bekannt, dass Endoskope zur Diagnose und Therapie von Erkrankungen eingesetzt werden. Für die Verwendung von Endoskopen sowie anderen chirurgischen Geräten ist es notwendig, dass diese chirurgischen Instrumente nach einem Einsatz bei einem Patienten aufbereitet, das heißt gereinigt und desinfiziert, werden müssen. Bei der Aufbereitung der chirurgischen Instrumente sind dabei gesetzliche sowie klinische Vorschriften streng einzuhalten, so dass die Bestandteile im Inneren und an der Oberfläche der chirurgischen Instrumente, insbesondere Endoskope, desinfiziert werden und frei von Keimen, Bakterien und so weiter sein müssen.

Aufbereitungsvorrichtungen von chirurgischen Instrumenten, insbesondere von Endoskopen, verfügen üblicherweise über Vorrichtungen, die sowohl die äußeren Oberflächen der chirurgischen Instrumente als auch die Kanäle sowie Kanalsysteme unter Verwendung von entsprechenden Flüssigkeiten derselben reinigen und desinfizieren. Hierzu sind üblicherweise Spülkreisläufe vorgesehen. Insbesondere bei der Innenkanalspülung eines chirurgischen Instruments, insbesondere eines Endoskops bzw. eines flexiblen Endoskops hängt das hygienische Ergebnis der Aufbereitung von den Durchflussmengen eines Aufbereitungsfluids ab.

Darüber hinaus ist zur Aufbereitung und Desinfektion von flexiblen Endoskopen unter der Bezeichnung ETD eine Aufbereitungsvorrichtung der Olympus Winter & Ibe GmbH, Hamburg, bekannt. In dieser werden durch maschinelle Reinigung sowie Desinfektion von, insbesondere von flexiblen, Endoskopen die Kanäle der Endoskope mit einem Aufbereitungsfluid bzw. von Spül-, Reinigungs- und/oder Desinfektionsflüssigkeiten durchspült.

Bei den bekannten Aufbereitungsmaschinen werden zur Dosierung der Desinfektionsmittel und Reinigungsmittel Schlauchpumpen eingesetzt, wobei die Schlauchpumpen die Aufbereitungsfluide aus einem Kanister oder dergleichen mittels einer Sauglanze entnehmen.

In US-A-3 749 288 ist ferner ein Flüssigkeitsspender für eine Spülmaschine, wie z.B. für einen Geschirrspüler, offenbart. Hierbei weist der Flüssigkeitsspender in einem Behälter eine Vorratskammer auf, wobei unterhalb der Vorratskammer eine Dosierkammer vorgesehen ist. Die Vorratskammer ist an der Unterseite der Kammer mit Öffnungen versehen, so dass die Flüssigkeit aus der Vorratskammer in die Dosierkammer überführt wird.

Darüber hinaus offenbart US-A-2009/0000648 ein Verfahren und eine Vorrichtung zur Aufbereitung eines medizinischen Instruments, insbesondere eines Handstücks. Hierbei sind mehrere Vorratsbehälter für ein Schmiermittel sowie für ein enzymatisches Reinigungsmittel und für ein Oxidationsmittel sowie Anschlüsse für eine Wasserversorgung und eine Druckluftversorgung mit einer Steuer-/Dosiereinrichtung verbunden, die mittels einer Computervorrichtung gesteuert wird.

Ausgehend von diesem Stand der Technik besteht die Aufgabe der Erfindung darin, eine einfache und wartungsfreie Dosierung von Aufbereitungsfluiden zur Reinigung und Desinfektion von chirurgischen Instrumenten, insbesondere Endoskopen, zu ermöglichen.

Gelöst wird diese Aufgabe durch eine Dosiereinrichtung für eine Aufbereitungsvorrichtung zum Aufbereiten von chirurgischen Instrumenten, insbesondere Endoskopen, mit einer vorbestimmten Dosierkammer zur Aufnahme eines Aufbereitungsfluids, insbesondere einer Reinigungsflüssigkeit oder einer Desinfektionsflüssigkeit, wobei die Dosierkammer einen Zulauf zum Einlassen des Aufbereitungsfluids in die Dosierkammer und einen Auslass zum Ablassen eines in die Dosierkammer eingebrachten Aufbereitungsfluids aufweist, wobei der Zulauf und der Auslass so dimensioniert sind, dass die Fließgeschwindigkeit des Aufbereitungsfluids im Auslass beim Ablassen des Aufbereitungsfluids aus der Dosierkammer größer als die Fließgeschwindigkeit des Aufbereitungsfluids im Zulauf beim Einbringen des Aufbereitungsfluids in die Dosierkammer ist, wobei der Zulauf und der Auslass so dimensioniert sind, dass eine Dosiergenauigkeit von weniger als ± 5% (< ± 5%) eingestellt ist.

Die Erfindung beruht auf dem Gedanken, dass bei der Dosiereinrichtung die Dosierkammer ein vorbestimmtes Volumen aufweist, in das das Aufbereitungsfluid aus einem Vorrat, vorzugsweise oberhalb der Dosierkammer, infolge von einer Gravitationsbefüllung entnommen und über den Zulauf in die Dosierkammer, vorzugsweise unterhalb des Vorrats, eingebracht wird, wobei insbesondere die Befüllzeit zur Befüllung der Dosierkammern sehr viel größer ist, als die Zeit für die Entleerung der Dosierkammer. Hierbei wird die Dosierkammer über einen längeren Zeitraum langsam bis zum maximalen Füllstand aus dem Vorrat mit dem Aufbereitungsfluid befüllt und danach aufgrund eines gegenüber dem Zulauf größeren Strömungsquerschnitt im Auslass innerhalb eines kurzen Zeitraumes, insbesondere schlagartig, entleert. Die Fließgeschwindigkeit beim Ablassen des Aufbereitungsfluids aus der Dosierkammer kann mindestens um den Faktor 2, 3, 4, 5, bis 10 oder 20 größer als die Fließgeschwindigkeit des Aufbereitungsfluids beim Befüllen der Dosierkammer im Zulauf sein. Dementsprechend ist die Befüllzeit, d.h. die Zeit zum Befüllen der anfangs leeren Dosierkammer bis zum vollständigen Füllstand mindestens um den Faktor 2, 3, 4, 5, bis 10 bzw. 20 größer als die Zeit zum Entleeren der Dosierkammer über den Auslass.

Hierbei wird eine Dosierung des Aufbereitungsfluids dadurch bereitgestellt, dass die Dosierkammer signifikant langsamer durch das nachlaufende Aufbereitungsfluid aus dem Behälter wieder befüllt wird, als die Entnahme bzw. die Entleerung der befüllten Dosierkammer durch den geöffneten Auslass erfolgt. Ist beispielsweise die Entnahme um den Faktor 100 schneller als die Befüllung, wird eine Dosiergenauigkeit von 1% erreicht.

Vorteilhafterweise sind der Zulauf und der Auslauf so dimensioniert, dass eine Dosiergenauigkeit von weniger als ± 5% (< ± 5%) eingestellt ist.

Ein weiterer Vorteil der Erfindung besteht darin, dass bei einer Aufbereitungsvorrichtung für chirurgische Instrumente der Einsatz von Schlauchpumpen oder anderen Pumpeneinrichtungen zur Entnahme von Aufbereitungsfluiden aus Behältern entbehrlich ist, so dass eine pumpenfreie Dosierung von Aufbereitungsfluiden für chirurgische Instrumente bereitgestellt wird.

Darüber hinaus zeichnet sich eine Weiterbildung dadurch aus, dass am Auslass ein Ventil vorgesehen ist, wobei insbesondere das Ventil beim Befüllen der Dosierkammer geschlossen ist oder beim Ablassen des Aufbereitungsfluids aus der Dosierkammer geöffnet ist.

Vorzugsweise ist weiterhin der Zulauf als ventilloser Zulauf ausgebildet, wobei der Zulauf mit dem mit Aufbereitungsfluid befüllten Behäiter verbunden oder verbindbar ist. Dadurch, dass das Aufbereitungsfluid aufgrund der Schwerkraft selbsttätig aus dem Behälter über den Zulauf in die Dosierkammer fließt, wird ein gravimetrisches Dosiersystem bereitgestellt. Hierbei befindet sich der Behälter mit dem Aufbereitungsfluid oberhalb der Dosierkammer.

Außerdem zeichnet sich eine Ausführungsform von der Dosiereinrichtung dadurch aus, dass wenigstens ein Kontrollfenster für die optische Kontrolle des Füllstands des Aufbereitungsfluids in der Dosierkammer vorgesehen ist.

Im Rahmen der Erfindung ist es dabei ebenfalls möglich, dass eine Füllstandsanzeige oder eine Füllstandssensorik für die Dosierkammer bzw. die Dosiereinrichtung vorgesehen ist, um den Befüllungsgrad in der Dosierkammer anzuzeigen und/oder zu erfassen. Eine derartige Kontrolleinrichtung kann beispielsweise mittels einer Lichtschranke oder dergleichen ausgebildet sein.

Darüber hinaus ist in einer Weiterbildung der Dosiereinrichtung vorgesehen, dass an der Dosierkammer bezogen auf die Fließrichtung des Aufbereitungsfluids zwischen dem, vorzugsweise oberen, Zulauf und dem, insbesondere unteren, Auslass zwei voneinander beabstandete Kontrollfenster vorgesehen sind. Hierbei wird mittels eines unteren Kontrollfensters, vorzugsweise im Bereich des Auslasses, eine optische Kontrolle möglich, ob beim Entleeren der Dosierkammer diese auch vollständig entleert ist.

Vorzugsweise ist der Zulauf mit einem Behälter für das Aufbereitungsfluid verbunden oder verbindbar, wobei insbesondere das Aufbereitungsfluid aus dem Behälter über den Zulauf in die Dosierkammer durch Schwerkraft selbsttägig einbringbar ist oder eingebracht wird. Hierbei wird der Auslass der Dosierkammer mit einem Einlass für ein Aufbereitungsfluid in einem Spülraum einer Aufbereitungsvorrichtung verbunden.

Ferner wird die Aufgabe gelöst durch eine Verwendung einer Dosiereinrichtung, wie voranstehend beschrieben, zur dosierten Abgabe eines Aufbereitungsfluids, insbesondere einer Reinigungsflüssigkeit oder einer Desinfektionsflüssigkeit, in den Spülraum einer Aufbereitungsvorrichtung zum Aufbereiten von chirurgischen Instrumenten, insbesondere Endoskopen.

Außerdem wird die Aufgabe gelöst durch eine Aufbereitungsvorrichtung zum Aufbereiten von chirurgischen Instrumenten, insbesondere Endoskopen, mit einem Spülraum zum Aufnehmen und Aufbereiten wenigstens eines chirurgischen Instruments, insbesondere Endoskops, und mit einer Dosiereinrichtung, wie voranstehend beschrieben, zum dosierten Abgeben eines Aufbereitungsfluids in den Spülraum. Zur Vermeidung von Wiederholungen wird auf die obigen Ausführungen ausdrücklich verwiesen.

Ferner wird die Aufgabe gelöst durch einen handhabbaren Vorratsbehälter, insbesondere Kanister, mit einem Tank für ein Aufbereitungsfluid zum Aufbereiten eines chirurgischen Instruments, insbesondere Endoskops, und mit einer voranstehend beschriebenen Dosiereinrichtung. Hierbei wird beispielsweise der Vorratsbehälter mit einer integrierten Dosiereinrichtung bereitgestellt, so dass die Dosierkammer des Vorratsbehälters an eine Aufbereitungsvorrichtung am Auslass angeschlossen wird.

Durch die Bereitstellung eines Vorratsbehälters mit einer integrierten Dosiereinrichtung bzw. Dosierkammer wird eine einfache Handhabung erreicht, wobei bei Austausch eines leeren Vorratsbehälters gegen einen mit einem Aufbereitungsfluid gefüllten Vorratsbehälter an der Aufbereitungsvorrichtung auch die Dosierkammer gemeinsam mit dem Vorratsbehälter ausgetauscht wird, so dass in Folge des Vorratsbehälterwechsels eine benutzte Dosierkammer des leeren Vorratsbehälters durch eine hygienisch reine Dosierkammer des vollen Vorratsbehälters ersetzt wird. Somit wird die Hygiene für die Aufbereitung von chirurgischen Instrumenten sowie Reinigung von Aufbereitungsvorrichtungen sowie deren Wartung auf einfache und effiziente Weise verbessert.

Ferner wird die Aufgabe gelöst durch ein Verfahren zum Aufbereiten eines chirurgischen Instruments, insbesondere Endoskops, mittels eines Aufbereitungsfluids in einem Spülraum, vorzugsweise in einem Spülraum einer Aufbereitungsvorrichtung, wobei unter Verbindung einer Dosiereinrichtung, wie voranstehend beschrieben, das Aufbereitungsfluid in den Spülraum der Aufbereitungsvorrichtung dosiert gegeben wird.

Weitere Merkmale der Erfindung werden aus der Beschreibung erfindungsgemäßer Ausführungsformen zusammen mit den Ansprüchen und der beigefügten Zeichnung ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von eines Ausführungsbeispiels unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

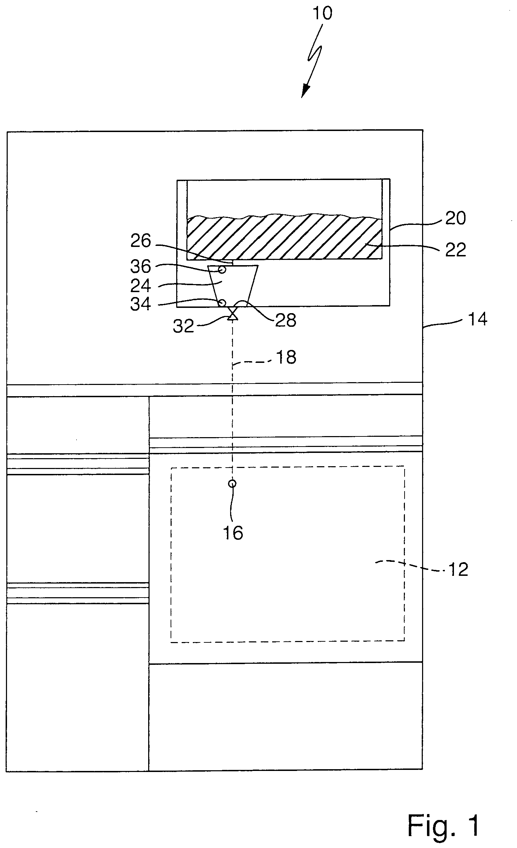

- Fig. 1: schematisch eine Ansicht einer Aufbereitungsvorrichtung zum Aufbereiten von chirurgischen Instrumenten, insbesondere von Endoskopen.

In Fig. 1 ist schematisch eine Aufbereitungsvorrichtung 10 für Endoskope in einer Frontansicht dargestellt. Zur Aufbereitung von chirurgischen Instrumenten weist dabei die Aufbereitungsvorrichtung ein Gehäuse 14 mit einem im Inneren des Gehäuses 14 ausgebildeten Aufnahmeraum 12 auf. In den als Spülraum ausgebildeten Aufnahmeraum 12 werden die zu reinigenden chirurgischen Instrumente, wie z.B. Endoskope, z.B. in Körben eingebracht. Eine Endoskopaufbereitungsvorrichtung ist beispielsweise unter der Bezeichnung ETD der Olympus Winter & Ibe GmbH, Hamburg, bekannt.

Die Aufbereitungsvorrichtung 10 weist ferner ein Bedienungs- und Anzeigenfeld sowie eine Trocknungseinrichtung zur Heißlufttrocknung von chirurgischen Instrumenten auf.

Ferner weist der Aufnahmeraum 12 einen Einlass 16 auf, der über eine Leitung 18 mit einem Vorratsbehälter 20 verbunden ist.

Der Vorratsbehälter 20 selbst ist oberhalb des Aufnahmeraums 12 in einem entsprechenden Fach oder dergleichen oder Halterung angeordnet. Der Vorratsbehälter 20 ist weiterhin mit einem Tank 22 zur Aufnahme eines Aufbereitungsfluids 22 ausgebildet. Hierbei können als Aufbereitungsfluid beispielsweise eine Reinigungsflüssigkeit oder eine Desinfektionsflüssigkeit sowie eine weitere Spülflüssigkeit in konzentrierter Form aufgenommen sein.

Darüber hinaus ist der Vorratsbehälter 20 mit einer Dosierkammer 24 ausgebildet, wobei die Dosierkammer 24 mit dem Tank 22 über einen Zulauf 26 verbunden ist. Die Dosierkammer 24 weist ein Volumen auf, das der Dosiermenge an Aufbereitungsflüssigkeit entspricht, die über den Einlass 16 in den Aufnahmeraum 12 in konzentrierter Form eingebracht wird. Der Tank 22 ist dabei oberhalb des Zulaufes 26 und der Dosierkammer 24 positioniert.

Aufgrund der Schwerkraft wird die Aufbereitungsflüssigkeit aus dem Tank 22 über den Zulauf 26 in die Dosierkammer 24 eingebracht, so dass die Dosierkammer 24 mit einem Aufbereitungsfluid befüllbar ist. Die Dosierkammer 24 weist an ihrer Unterseite einen mit der Leitung 18 verbundenen Auslass 28 auf, über den nach Befüllung der Dosierkammer 24 mit einem Aufbereitungsfluid das Aufbereitungsfluid über den Auslass 28 über die an den Auslass 28 angeschlossene Leitung 18 abfließt. Hierbei ist am Auslass 28 ein Ventil 32 angeordnet.

Der obere Zulauf 26 und der untere Auslass 28 sind erfindungsgemäß so ausgebildet, dass die Fließgeschwindigkeit des Aufbereitungsfluids im Zulauf 26 sehr viel kleiner ist als beim Ablassen des Aufbereitungsfluids aus der befüllten Dosierkammer 24, wodurch das Aufbereitungsfluid langsam zur Befüllung der Dosierkammer 24 in diese aus dem Tank 22 über den Zulauf 26 fließt, während nach Befüllung der Dosierkammer 24 das Aufbereitungsfluid schneller aus der Dosierkammer 24 über den Auslass 28 fließt, wodurch eine vorbestimmte Menge an Aufbereitungsfluid über die Leitung 18 und den Einlass 16 in den Aufnahmeraum 12 fließt.

Zur Überwachung des Füllstands der Dosierkammer 24 bei Befüllung der Dosierkammer 24 ist im unteren Bereich sowie im oberen Bereich ein Kontrollfenster 34, 36 angeordnet. Hierdurch kann auf optische Weise der Füllstand des Aufbereitungsfluids in der Dosierkammer 24 erfasst werden.

Alle genannten Merkmale, auch die der Zeichnung allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 10: Aufbereitungsvorrichtung
- 12: Aufnahmeraum
- 14: Gehäuse
- 16: Einlass
- 18: Leitung
- 20: Vorratsbehälter
- 22: Tank
- 24: Dosierkammer
- 26: Zulauf
- 28: Auslass
- 32: Ventil
- 34: Kontrollfenster
- 36: Kontrollfenster

## Patentansprüche

1. Dosiereinrichtung für eine Aufbereitungsvorrichtung (10) zum Aufbereiten von chirurgischen Instrumenten, insbesondere Endoskopen, mit einer vorbestimmten Dosierkammer (24) zur Aufnahme eines Aufbereitungsfluids, insbesondere einer Reinigungsflüssigkeit oder einer Desinfektionsflüssigkeit, wobei die Dosierkammer (24) einen Zulauf (26) zum Einlassen des Aufbereitungsfluids in die Dosierkammer (24) und einen Auslass (28) zum Ablassen eines in die Dosierkammer (24) eingebrachten Aufbereitungsfluids aufweist, wobei der Zulauf (26) und der Auslass (28) so dimensioniert sind, dass die Fließgeschwindigkeit des Aufbereitungsfluids im Auslass (28) beim Ablassen des Aufbereitungsfluids aus der Dosierkammer (24) größer als die Fließgeschwindigkeit des Aufbereitungsfluids im Zulauf (26) beim Einbringen des Aufbereitungsfluids in die Dosierkammer (24) ist, wobei der Zulauf (26) und der Auslass (28) so dimensioniert sind, dass eine Dosiergenauigkeit von weniger als ± 5% (< ± 5%) eingestellt ist.

2. Dosiereinrichtung nach Anspruch 1, **dadurch gekennzeichnet, dass** am Auslass (28) ein Ventil (32) vorgesehen ist, wobei insbesondere das Ventil (32) beim Befüllen der Dosierkammer (24) geschlossen ist oder beim Ablassen des Aufbereitungsfluids aus der Dosierkammer (24) geöffnet ist.

3. Dosiereinrichtung nach Anspruch 1 oder 2, **dadurch gekennzeichnet, dass** der Zulauf (26) als ventilloser Zulauf (26) ausgebildet ist.

4. Dosiereinrichtung nach einem der Ansprüche 1 bis 3, **dadurch gekennzeichnet, dass** wenigstens ein Kontrollfenster (34, 36) für die optische Kontrolle des Füllstands des Aufbereitungsfluids in der Dosierkammer (24) vorgesehen ist.

5. Dosiereinrichtung nach Anspruch 4, **dadurch gekennzeichnet, dass** an der Dosierkammer (24) bezogen auf die Fließrichtung des Aufbereitungsfluids zwischen dem Zulauf (26) und dem Auslass (28) zwei voneinander beabstandete Kontrollfenster (34, 36) vorgesehen sind.

6. Dosiereinrichtung nach einem der Ansprüche 1 bis 5, **dadurch gekennzeichnet, dass** der Zulauf (26) mit einem Behälter (20) für das Aufbereitungsfluid verbunden oder verbindbar ist, wobei insbesondere das Aufbereitungsfluid aus dem Behälter über den Zulauf (26) in die Dosierkammer (24) durch Schwerkraft selbsttätig einbringbar ist oder eingebracht wird.

7. Verwendung einer Dosiereinrichtung nach einem der Ansprüche 1 bis 6 zur dosierten Abgabe eines Aufbereitungsfluids, insbesondere einer Reinigungsflüssigkeit oder einer Desinfektionsflüssigkeit, in den Spülraum (12) einer Aufbereitungsvorrichtung (10) zum Aufbereiten von chirurgischen Instrumenten, insbesondere Endoskopen.

8. Aufbereitungsvorrichtung (10) zum Aufbereiten von chirurgischen Instrumenten, insbesondere Endoskopen, mit einem Spülraum zum Aufnehmen und Aufbereiten wenigstens eines chirurgischen Instruments, insbesondere Endoskops, und mit einer Dosiereinrichtung nach einem der Ansprüche 1 bis 6 zum dosierten Abgeben eines Aufbereitungsfluids in den Spülraum.

9. Vorratsbehälter (20), insbesondere Kanister, mit einem Tank (22) für ein Aufbereitungsfluid zum Aufbereiten eines chirurgischen Instruments, insbesondere Endoskops, und mit einer Dosiereinrichtung nach einem der Ansprüche 1 bis 6.

10. Verfahren zum Aufbereiten eines chirurgischen Instruments, insbesondere Endoskops, mittels eines Aufbereitungsfluids in einem Spülraum (12), vorzugsweise in einem Spülraum (12) einer Aufbereitungsvorrichtung (10), wobei unter Verwendung einer Dosiereinrichtung nach einem der Ansprüche 1 bis 6 das Aufbereitungsfluid in den Spülraum dosiert gegeben wird.

## Claims

1. A metering device for a reprocessing apparatus (10) for reprocessing surgical instruments, in particular endoscopes, with a predetermined metering chamber (24) for accommodating a reprocessing fluid, particularly a cleaning liquid or a disinfecting liquid, wherein the metering chamber (24) comprises an inlet (26) for letting the reprocessing fluid into the metering chamber (24) and an outlet (28) for letting out a reprocessing fluid introduced into the metering chamber (24), wherein the inlet (26) and the outlet (28) are dimensioned in such a way that the flow velocity of the reprocessing fluid in the outlet (28) while the reprocessing fluid is being let out of the metering chamber (24) is greater than the flow velocity of the reprocessing fluid in the inlet (26) while the reprocessing fluid is being introduced into the metering chamber (24), wherein the inlet (26) and the outlet (28) are dimensioned so that a metering accuracy of less than ± 5% (< ± 5%) is set.

2. The metering device according to claim 1, **characterized in that** a valve (32) is provided on the outlet (28), wherein in particular the valve (32) is closed during the filling of the metering chamber (24) or is open during the letting of the reprocessing fluid out of the metering chamber (24).

3. The metering device according to claim 1 or 2, **characterized in that** the inlet (26) is designed as a valveless inlet (26).

4. The metering device according to one of claims 1 to 3, **characterized in that** at least one control window (34, 36) is provided for the optical monitoring of the fill level of the reprocessing fluid in the metering chamber (24).

5. The metering device according to claim 4, **characterized in that** two control windows (34, 36) spaced apart from each other are provided on the metering chamber (24) between the inlet (26) and the outlet (28) in relation to the flow direction of the reprocessing fluid.

6. The metering device according to one of claims 1 to 5, **characterized in that** the inlet (26) is connected or connectable to a container (20) for the reprocessing fluid, wherein in particular the reprocessing fluid is automatically introducible or introduced into the metering chamber (24) from the container via the inlet (26) through gravity.

7. A use of a metering device according to one of claims 1 to 6 for metered delivery of a reprocessing fluid, in particular a cleaning liquid or a disinfecting liquid, into the washing area (12) of a reprocessing device (10) for reprocessing surgical instruments, particularly endoscopes.

8. A reprocessing apparatus (10) for reprocessing surgical instruments, particularly endoscopes, with a washing area for accommodating and reprocessing at least one surgical instrument, in particular an endoscope, and with a metering device according to one of claims 1 to 6 for the metered delivery of a reprocessing fluid into the washing area.

9. A reservoir (20), in particular a canister, with a tank (22) for a reprocessing fluid for reprocessing a surgical instrument, in particular an endoscope, and with a metering device according to one of claims 1 to 6.

10. A method for reprocessing a surgical instrument, in particular an endoscope, by means of a reprocessing fluid in a washing area (12), preferably in a washing area (12) of a reprocessing apparatus (10), wherein, upon use of a metering device according to one of claims 1 to 6, the reprocessing fluid is delivered to the washing area in a metered manner.

## Revendications

1. Système de dosage pour un dispositif de traitement (10) destiné à traiter des instruments chirurgicaux, en particulier des endoscopes, ayant une chambre de dosage prédéfinie (24) pour recevoir un fluide de traitement, en particulier un liquide de nettoyage ou un liquide de désinfection, dans lequel la chambre de dosage (24) comporte une entrée (26) pour faire entrer le fluide de traitement dans la chambre de dosage (24) et une sortie (28) pour faire sortir un fluide de traitement introduit dans la chambre de dosage (24), dans lequel l'entrée (26) et la sortie (28) sont dimensionnées de sorte que la vitesse d'écoulement du fluide de traitement dans la sortie (28) lors de l'évacuation du fluide de traitement à partir de la chambre de dosage (24) est supérieure à la vitesse d'écoulement du fluide de traitement dans l'entrée (26) lors de l'introduction du fluide de traitement dans la chambre de dosage (24), dans lequel l'entrée (26) et la sortie (28) sont dimensionnées de sorte qu'une précision de dosage est réglée à moins de ± 5 % (< ± 5 %).

2. Système de dosage selon la revendication 1, **caractérisé en ce qu'**un clapet (32) est prévu sur la sortie (28), dans lequel le clapet (32) est en particulier fermé lors du remplissage de la chambre de dosage (24) ou est ouvert lors de l'évacuation du fluide de traitement à partir de la chambre de dosage (24).

3. Système de dosage selon la revendication 1 ou la revendication 2, **caractérisé en ce que** l'entrée (26) est formée comme une entrée (26) sans soupape.

4. Système de dosage selon l'une des revendications 1 à 3, **caractérisé en ce qu'**au moins une fenêtre de contrôle (34, 36) est prévue pour le contrôle optique du niveau de remplissage du fluide de traitement dans la chambre de dosage (24).

5. Système de dosage selon la revendication 4, **caractérisé en ce que** deux fenêtres de contrôle (34, 36) espacées l'une de l'autre sont prévues sur la chambre de dosage (24) par rapport à la direction d'écoulement du fluide de traitement entre l'entrée (26) et la sortie (28).

6. Système de dosage selon l'une des revendications 1 à 5, **caractérisé en ce que** l'entrée (26) est reliée ou peut être reliée à un conteneur (20) pour le fluide de traitement, dans lequel le fluide de traitement peut être en particulier introduit ou est introduit automatiquement, par gravité, à partir du conteneur dans la chambre de dosage (24), par l'intermédiaire de l'entrée (26).

7. Utilisation d'un système de dosage selon l'une des revendications 1 à 6 pour la distribution dosée d'un fluide de traitement, en particulier d'un liquide de nettoyage ou d'un liquide de désinfection, dans la zone de lavage (12) d'un dispositif de traitement (10) pour traiter des instruments chirurgicaux, en particulier des endoscopes.

8. Dispositif de traitement (10) pour traiter des instruments chirurgicaux, en particulier des endoscopes, ayant une zone de lavage pour recevoir et traiter au moins un instrument chirurgical, en particulier un endoscope, et comportant un système de dosage selon l'une des revendications 1 à 6 pour une distribution dosée d'un fluide de traitement dans la zone de lavage.

9. Conteneur de stockage (20), en particulier un bidon, comportant un réservoir (22) pour un fluide de traitement destiné à traiter un instrument chirurgical, en particulier un endoscope, et ayant un système de dosage selon l'une des revendications 1 à 6.

10. Procédé pour traiter un instrument chirurgical, en particulier un endoscope, au moyen d'un fluide de traitement dans une zone de lavage (12), de préférence dans une zone de lavage (12) d'un dispositif de traitement (10), dans lequel le fluide de traitement est distribué de manière dosée dans la zone de lavage, en utilisant un système de dosage selon l'une des revendications 1 à 6.
